# EUROPÄISCHE PATENTSCHRIFT

(11) **EP 1 203 763 B1**
(45) Veröffentlichungstag und Bekanntmachung des Hinweises auf die Patenterteilung: **18.11.2009**
(21) Anmeldenummer: 01122856.6
(22) Anmeldetag: 24.09.2001
(51) Int. Cl.: C07C 233/69, C07C 231/02

(54) **Verfahren zur Herstellung von Beta-Hydroxyalkylamiden**
Process for the synthesis of beta-hydroxyalkylamides
Procédé pour la fabrication de béta-hydroxyalkylamides

(30) Priorität: 26.10.2000 DE 10053194
(43) Veröffentlichungstag der Anmeldung: 08.05.2002
(73) Patentinhaber: EMS-Chemie AG, 7013 Domat/Ems (CH)
(72) Erfinder: Kaplan, Andreas, 7000 Chur (CH); Gisler, René, 7000 Chur (CH)
(74) Vertreter: Pfenning, Meinig & Partner GbR

(56) Entgegenhaltungen:
- EP-A- 0 473 380
- DE-A- 19 823 925

## Beschreibung

Die Erfindung betrifft ein Verfahren zur Herstellung von β-Hydroxyalkylamiden.

β-Hydroxyalkylamide besitzen große Bedeutung als Zwischenprodukte und als Vernetzer für Polymere. Gewöhnlicherweise werden β-Hydroxyalkylamide durch Aminolyse von Alkylestern mit β-Aminalkoholen in Gegenwart von basischen Katalysatoren hergestellt. Die Isolierung und Reinigung der β-Hydroxyalkylamide erfolgt dabei entweder durch Kristallisation in einem Lösemittel oder speziell bei festen β-Hydroxyalkylamiden lösemittelfrei in einem sogenannten Slurry-Verfahren. Ein derartiges Verfahren ist in der US 5,101,073 sowie in der EP 0 473 380 B1 offenbart. Das Slurry-Verfahren beruht darauf, daß die bei der Herstellung der β-Hydroxyalkylamide ablaufende Gleichgewichtsreaktion in Richtung des gewünschten Endproduktes dadurch verschoben wird, daß durch Tempern in einem bestimmten Temperaturbereich das gewünschte β-Hydroxyalkylamid aus der Schmelze ausfällt und dadurch die Schmelze kristallisiert. Nachteilig bei diesem Verfahren ist der Einsatz von äquimolaren Mengen Alkylester und β-Hydroxyalkylamid.

Ein Verfahren zur Herstellung von β-Hydroxyalkylamiden ist in der DE 198 23 925 beschrieben. Bei diesem Verfahren wird in Abwesenheit von Lösemitteln der Ester mit den Aminalkoholen in Gegenwart von basischen Katalysatoren umgesetzt.

Aus der EP 0 473 380 A1 ist ein weiteres Verfahren zur Herstellung von β-Hydroxyalkylamiden bekannt, bei dem ebenfalls von Alkylestern ausgegangen wird.

Aus der EP-A-322 834 ist ein Pulverlack bekannt der einen Polyester und β-Hydroxyalkylamide als Vernetzer (Härter) enthält. Mit der darin beschriebenen Formulierung lassen sich entsprechende Beschichtungen mit guten Eigenschaften herstellen.

Aufgrund der großen Bedeutung der β-Hydroxyalkylamide als Zwischenprodukt, insbesondere als Vernetzer für Polyesterpulverlacke wie in der EP-A-322 834 offenbart, besteht in jüngster Zeit ein großes Interesse an neuartigen β-Hydroxyalkylamiden.

Ausgehend hiervon ist es die Aufgabe der vorliegenden Erfindung ein neues Verfahren zur Herstellung von β-Hydroxyalkylamiden anzugeben.

Die Aufgabe wird durch die kennzeichnenden Merkmale des Patentanspruches 1 gelöst. Die Unteransprüche zeigen vorteilhafte Weiterbildungen auf.

Die mit dem Verfahren hergestellten β-Hydroxyalkylamide sind durch die allgemeine Formel I 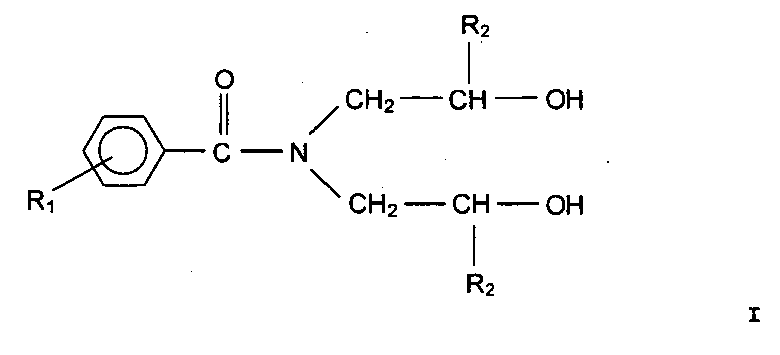 definiert. In der Formel 1 bedeuten R₁ Wasserstoff oder ein lineares oder verzweigtes C₁ bis C₁₀-Alkyl.
R₂ ist ein lineares oder verzweigtes C₁ bis C₅-Alkyl. Die β-Hydroxyalkylamide zeichnen sich insbesondere durch das zur OH-Gruppe benachbarte substituierte Kohlenstoffatom aus. Bei den β-Hydroxyalkylamiden ist hier der Rest R₂ angeordnet.

Bevorzugt ist das β-Hydroxyalkylamid so aufgebaut, daß der Rest R₁ H, tert-Butyl, Isopropyl oder Pentyl ist und in Parastellung zur CO-Gruppe angeordnet ist.

Eine ganz besonders bevorzugte Ausführungsform ist dadurch gekennzeichnet, daß der Rest R₁ Wasserstoff und der Rest R₂ Methyl ist.

Die erfindungsgemäße Herstellung dieser β-Hydroxyalkylamide erfolgt in der Weise, daß ein Carbonsäurederivat der allgemeinen Formel II 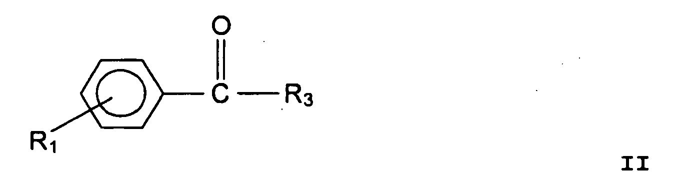 mit einem Alkanolamin der allgemeinen Formel III 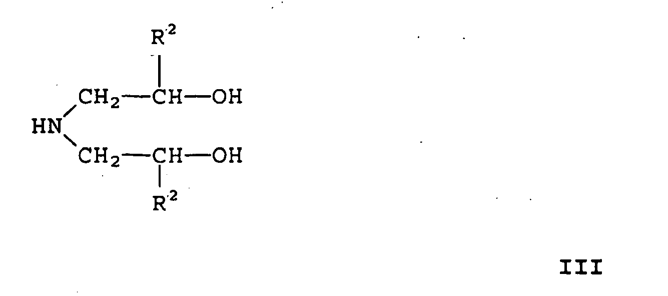 umgesetzt wird. Die Reste R₁ und R₂ besitzen dabei die vorstehend angegebene Bedeutung. Der Rest R₃ ist dabei ein Halogen, bevorzugt Chlor.

Die Umsetzung des Carbonsäurederivates und des Alkanolamins erfolgt bevorzugt in einer Lösung. Als bevorzugte Lösungsmittel sind zu nennen:

Aromatische Kohlenwasserstoffe wie Benzol, Toluol oder Xylol. Gut geeignet sind auch Ether wie Diethylether oder Mischungen der genannten Lösungsmittel.

Wesentlich beim erfindungsgemäßen Verfahren ist, daß das Carbonsäurederivat der allgemeinen Formel II und das Alkanolamin der allgemeinen Formel III unter kräftigem Rühren umgesetzt wird. Das Alkanolamin wird dabei in bevorzugter Weise vorgelegt und das Carbonsäurederivat dann unter kräftigem Rühren zugetropft.

Die Umsetzung erfolgt bei -10 bis 25°C, bevorzugt bei 0 bis 10 °C. Die Reaktionszeit liegt normalerweise bei 0,5 bis 5 Stunden. Eine Zeitspanne von 2 Stunden ist am günstigsten.

Das mit dem erfindungsgemäßen Verfahren hergestellte β-Hydroxyalkylamid ist besonders geeignet als Vernetzer für Polymere. Besonders bevorzugt wird das β-Hydroxyalkylamid als Vernetzer für Pulverlacke mit Polyestern oder Acrylaten als Polymer eingesetzt.

Grundsätzlich kann das β-Hydroxyalkylamid analog den β-Hydroxyalkylamiden, wie sie in der EP-A-322 834 genannt sind, als Vernetzer (Härter) eingesetzt werden. Die erfindungsgemäß hergestellten β-Hydroxyalkylamide können auch in Kombination mit anderen Härtern verwendet werden. Als Beispiel ist hier der Härter der unter dem Namen "PRIMID^{®}" von der Firma EMS-CHEMIE AG, Domat/Ems, vertrieben wird, zu nennen. PRIMID^{®} ist N,N,N',N' 2-Hydroxyethyladipamid.

Die Erfindung wird nachfolgend anhand eines Herstellungsbeispiels, bei der R₁ H und R₂ CH₃ ist, näher erläutert:

### Herstellungsbeispiel:

In einem 1 1 Vierhalsrundkolben mit Rührer, Tropftrichter, Thermometer und Rückflusskühler werden 44,16 g (0,32 mol) wasserfreies K₂CO₃, 42,56 g (0,32 mol) Diisopropanolamin, 160 ml Wasser und 160 ml Diethylether vorgelegt. In den Tropftrichter werden 44,96 g (0,32 mol) Benzoylchlorid eingewogen und in 160 ml Toluol gelöst. Diese Lösung wird nun unter kräftigem Rühren innerhalb von 2 Stunden in den Reaktionskolben eingetropft. Während der Zugabe wird die Temperatur des Reaktionsgemisches zwischen 0 und 5 °C gehalten. Nach der Zugabe wird noch während 30 Minuten bei 0 bis 5°C weiter gerührt. Anschließend wird das Eisbad entfernt und das Gemisch noch während 50 Minuten bei Raumtemperatur gerührt.

Der sich während der Reaktion gebildete Niederschlag wird abfiltriert, zweimal mit 35 ml Toluol und anschließend mit dreimal 30 ml Diethylether gewaschen und dann bei 50 °C unter Vakuum getrocknet. Das getrocknete Rohprodukt (74,28 g) wird in Benzol aufgekocht (25%ige Lösung), die unlöslichen Anteile abfiltriert und das Produkt bei Raumtemperatur kristallisiert. Nach der Filtration und Trocknung erhält man 51,80 g (68%) N,N-Bis-(2-Hydroxyisopropyl)-benzamid, Schmelzpunkt 103 °C. Elementaranalyse: Berechnet für C₁₃H₁₉NO₃ : C = 65.82%; H 8,02%; N = 5,91%; O = 20,25%. Gefunden: C = 66.25%; H = 8.09%; N = 5.83%; O = 19.83%.

## Patentansprüche

1. Verfahren zur Herstellung von β-Hydroxyalkylamid der allgemeinen Formel I 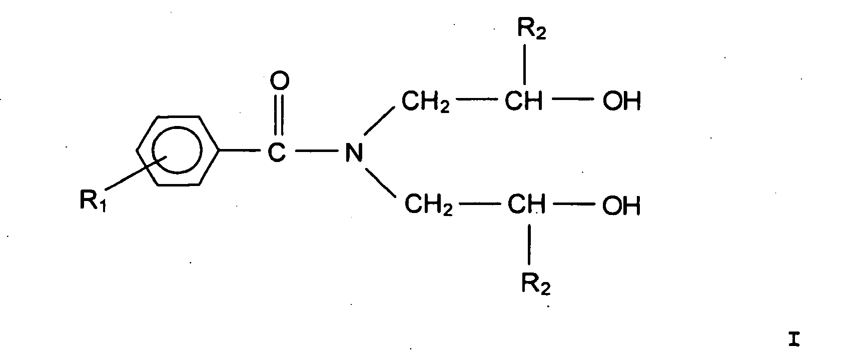 worin R₁ H oder ein lineares oder verzweigtes C₁ bis C₁₀-Alkyl und R₂ ein linear oder verzweigtes C₁ bis C₅-Alkyl ist,
**dadurch gekennzeichnet,**
**dass** ein Carbonsäurederivat der allgemeinen Formel II 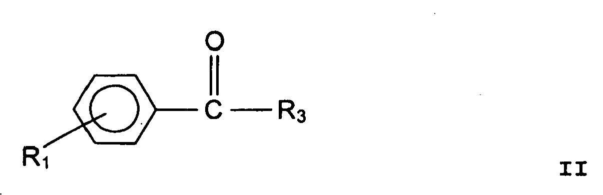 worin R₃ Halogen bedeutet, mit einem Alkanolamin der Formel III 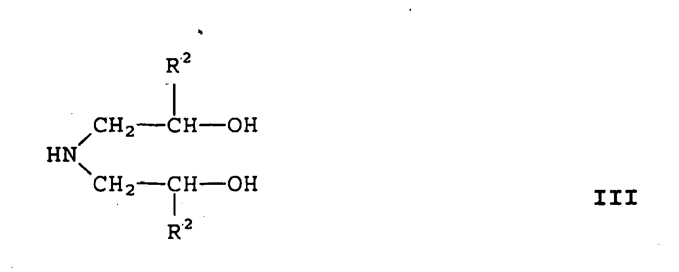 umgesetzt wird.

2. Verfahren nach Anspruch 1, **dadurch gekennzeichnet, dass** in der allgemeinen Formel I R₁ H, t-Butyl, i-Propyl oder Pentyl und in Parastellung zur CO-Gruppe angeordnet ist.

3. Verfahren nach Anspruch 2, **dadurch gekennzeichnet, dass** R₁ H und R₂ CH₃ ist.

4. Verfahren nach einem der Ansprüche 1 bis 3, **dadurch gekennzeichnet, daß** die Umsetzung mit dem Alkanolamin der Formel III bei -10 bis 25 °C durchgeführt wird.

5. Verfahren nach einem der Ansprüche 1 bis 4, **dadurch gekennzeichnet, daß** Benzoylchlorid als Carbonsäurederivat und Diisopropanolamin als Alkanolamin eingesetzt wird.

6. Verfahren nach einem der Ansprüche 1 bis 5, **dadurch gekennzeichnet, daß** das Carbonsäurederivat der allgemeinen Formel II und das Alkanolamin der Formel III in einem Lösungsmittel unter kräftigem Rühren umgesetzt wird.

7. Verfahren nach einem der Ansprüche 1 bis 6, **dadurch gekennzeichnet, daß** als Lösungsmittel aromatische Kohlenwasserstoffe wie Benzol, Toluol oder Xylol und/oder Ether eingesetzt werden.

8. Verfahren nach einem der Ansprüche 4 bis 7, **dadurch gekennzeichnet, daß** das Alkanolamin vorgelegt und das Carbonsäurederivat unter kräftigem Rühren zugegeben wird.

## Claims

1. Process for the production of β-hydroxyalkylamide having the general Formula I 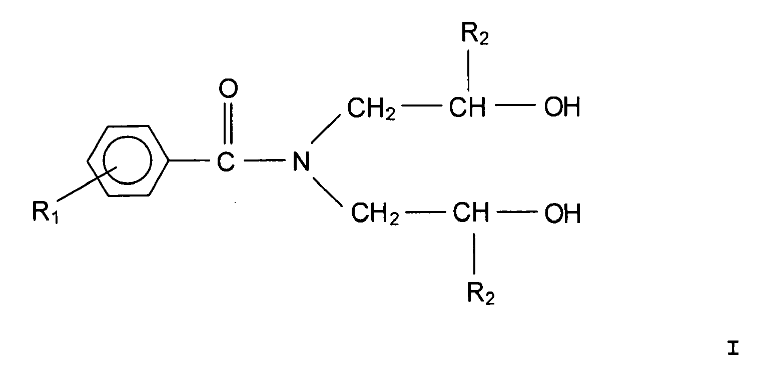 where R₁ is H or a linear or branched C₁ to C₁₀ alkyl and R₂ is a linear or branched C₁ to C₅ alkyl,
**characterized in that** a carboxylic acid derivative having the general Formula II: 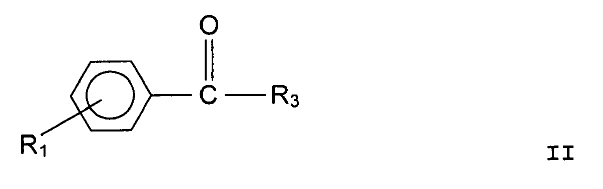 where R₃ is halogen, is reacted with an alkanol amine having the general Formula III:

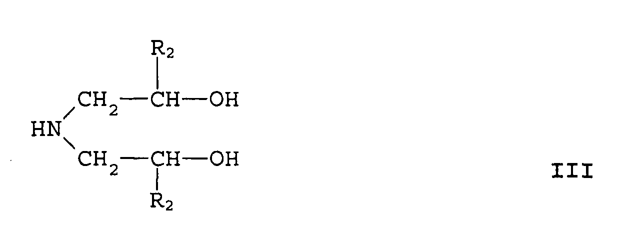

2. Process as claimed in claim 1,
**characterized by** the fact that R₁ is H, t-butyl, i-propyl or pentyl and is located in the para-position to the CO group.

3. Process as claimed in claim 2,
**characterized by** the fact that R₁ is H and R₂ is CH₃.

4. Process as claimed in one of the claims 1 to 3,
**characterized by** the fact that the alkanol amine having the general Formula III is reacted at -10 to 25°C.

5. Process as claimed in one of the claims 1 to 4,
**characterized by** the fact that benzoyl chloride is used as the carboxylic acid derivative and diisopropanol amine as the alkanol amine.

6. Process as claimed in at least one of the claims 1 to 5,
**characterized by** the fact that the carboxylic acid derivative having the general Formula II and the alkanol amine having the general Formula III are reacted in a solvent with vigorous agitation or stirring.

7. Process as claimed in at least one of the claims 1 to 6,
**characterized by** the fact that aromatic hydrocarbons such as benzene, toluene or xylene and/or ether are used as the solvent.

8. Process as claimed in at least one of the claims 4 to 7,
**characterized by** the fact that the alkanol amine is presented first and the carboxylic acid derivative is added with vigorous agitation or stirring.

## Revendications

1. Procédé de fabrication d'un β-hydroxyalkylamide de formule générale 1 : 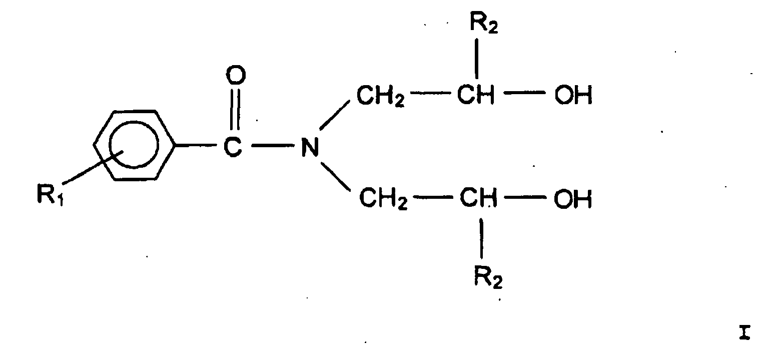 dans laquelle R₁ représente l'hydrogène ou un alkyle en C₁-C₁₀ linéaire ou ramifié et R₂ représente un alkyle en C₁-C₅ linéaire ou ramifié,
**caractérisé en ce que** :
on fait réagir un dérivé d'acide carboxylique de formule générale II : 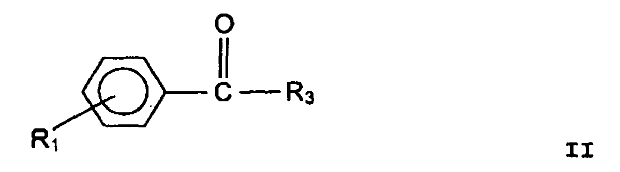
dans laquelle R₃ représente un halogène, avec une alcanolamine de formule III :

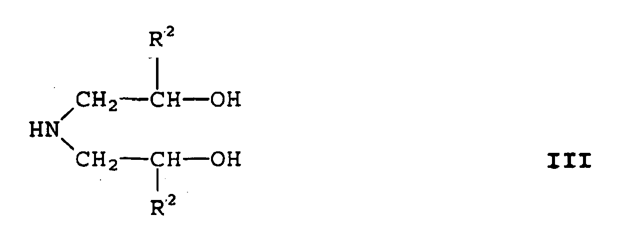

2. Procédé selon la revendication 1, **caractérisé en ce que** dans la formule générale I, R₁ représente l'hydrogène, un groupement t-butyle, i-propyle ou pentyle, et est placé en position para par rapport au groupement CO.

3. Procédé selon la revendication 2, **caractérisé en ce que** R₁ représente l'hydrogène et R₂ représente un groupement CH₃.

4. Procédé selon l'une quelconque des revendications 1 à 3, **caractérisé en ce que** la réaction avec l'alcanolamine de formule III est réalisée à une température de -10 à 25°C.

5. Procédé selon l'une quelconque des revendications 1 à 4, **caractérisé en ce que** l'on utilise du chlorure de benzoyle comme dérivé d'acide carboxylique et de la diisopropanolamine comme alcanolamine.

6. Procédé selon l'une quelconque des revendications 1 à 5, **caractérisé en ce que** l'on fait réagir le dérivé d'acide carboxylique de formule générale II et l'alcanolamine de formule III dans un solvant sous une agitation vigoureuse.

7. Procédé selon l'une quelconque des revendications 1 à 6, **caractérisé en ce que** l'on utilise, comme solvant, des hydrocarbures aromatiques, tels que le benzène, le toluène ou le xylène, et/ou des éthers.

8. Procédé selon l'une quelconque des revendications 4 à 7, **caractérisé en ce que** l'on incorpore l'alcanolamine et **en ce que** l'on ajoute le dérivé d'acide carboxylique sous agitation vigoureuse.
